# EUROPEAN PATENT APPLICATION

(11) **EP 4 818 143 A1**
(43) Date of publication of application: **07.10.2026**
(21) Application number: 24897675.5
(22) Date of filing: 29.11.2024
(51) Int. Cl.: C07C 1/26, C07B 61/00, C07C 15/56, C07C 41/30, C07C 43/205, C07C 45/70, C07C 49/83, C07C 67/343, C07C 69/76, C07D 471/04

(54) **BIARYL COMPOUND PRODUCTION METHOD**

(30) Priority: 30.11.2023 JP 2023202640
(71) Applicant: FUJIFILM Corporation, Tokyo 106-8620 (JP); FUJIFILM Wako Pure Chemical Corporation, Osaka 540-8605 (JP)
(72) Inventor: HITOMI, Seiichi, Ashigarakami-gun, Kanagawa 258-8577 (JP); WADA, Kenji, Ashigarakami-gun, Kanagawa 258-8577 (JP)
(74) Representative: HGF
(86) International application number: PCT/JP2024/042256
(87) International publication number: WO 2025/115991

(57) **Abstract**

A method for producing a biaryl compound includes: generating a benzyne compound from a precursor of the benzyne compound; subjecting the benzyne compound and a furan compound to a Diels-Alder reaction to generate a fused ring compound including a 1,4-epoxy-1,4-dihydrobenzene ring; and subjecting the fused ring compound and a halogenated aryl compound to a cross-coupling reaction to generate a biaryl compound.

## Description

### BACKGROUND OF THE INVENTION

### 1. Field of the Invention

The present invention relates to a method for producing a biaryl compound.

### 2. Description of the Related Art

In order to obtain a biaryl compound by introducing a naphthalene ring into a halogenated aryl compound, it has been known that a cross-coupling reaction is performed using the halogenated aryl compound and a naphthaleneboronic acid compound as reaction substrates. For example, WO2021/078217A discloses that an aromatic diazonium salt generated by diazotizing 5-bromoanthranilic acid is subjected to a Diels-Alder reaction with furan to obtain 6-bromo-1,4-epoxy-1,4-dihydronaphthalene, 2,8-dibromonaphthacene is obtained using this 6-bromo-1,4-epoxy-1,4-dihydronaphthalene as a reaction substrate, and this 2,8-dibromonaphthacene is subjected to a cross-coupling reaction with 1-naphthylboronic acid to obtain 2,8-dinaphthylnaphthacene which is a biaryl compound. However, the 1-naphthylboronic acid and the like (fused polycyclic aromatic boronic acid compounds) used in the above-described coupling reaction are expensive, and the production of the biaryl compound by the above-described reactions is restricted in terms of cost reduction.

In the above-described Diels-Alder reaction, the aromatic diazonium salt generated from 5-bromoanthranilic acid functions as a precursor of an o-benzyne compound (a compound that generates an o-benzyne compound). That is, in WO2021/078217A, the brominated o-benzyne generated by heating the above-described aromatic diazonium salt to 70°C undergoes the Diels-Alder reaction with furan, and thus the above-described 6-bromo-1,4-epoxy-1,4-dihydronaphthalene is generated.

Precursors of the o-benzyne compound itself are widely known. In addition to the aromatic diazonium salt generated from an anthranilic acid compound such as 5-bromoanthranilic acid described above, for example, a monohalogenobenzene compound, an o-dihalogenobenzene compound, a trifluoromethanesulfonic acid-2-(halogeno)phenyl compound, a trifluoromethanesulfonic acid-2-(trimethylsilyl)phenyl compound, and the like are used as the precursors of the o-benzyne compound in various Diels-Alder reactions.

### SUMMARY OF THE INVENTION

An object of the present invention is to provide a method for producing a biaryl compound, the method including obtaining a target biaryl compound without using, as a reaction substrate, an expensive reagent such as a fused polycyclic aromatic boronic acid compound, in a case of introducing the fused polycyclic aromatic structure into a halogenated aryl compound to obtain the target biaryl compound.

In view of the above-described problems, the present inventors have conducted intensive studies. As a result, the present inventors have found that, by performing a cross-coupling reaction between a fused ring compound including a 1,4-epoxy-1,4-dihydrobenzene ring, which is obtained by a Diels-Alder reaction between an o-benzyne compound and a furan compound, and a halogenated aryl compound, the fused ring compound is bonded, at a specific site, to a halogen-bonded site of the halogenated aryl compound, and then a conjugated structure is formed through cleavage of an epoxy group, thereby obtaining a target biaryl compound.

The invention has been further studied based on these findings, and has been completed.

The above-described objects of the present invention have been achieved by the following methods.
[1] A method for producing a biaryl compound, comprising:
   generating an o-benzyne compound from a precursor of the o-benzyne compound;
   subjecting the o-benzyne compound and a furan compound to a Diels-Alder reaction to generate a fused ring compound including a 1,4-epoxy-1,4-dihydrobenzene ring; and
   subjecting the fused ring compound and a halogenated aryl compound to a cross-coupling reaction to generate a biaryl compound.
[2] The method for producing a biaryl compound according to [1], in which the precursor of the o-benzyne compound is an aromatic diazonium salt.
[3] The method for producing a biaryl compound according to [1] or [2], in which the method includes diazotizing an anthranilic acid compound under an acidic condition to obtain an aromatic diazonium salt that is the precursor of the o-benzyne compound, and adding a base to a reaction system for the Diels-Alder reaction.
[4] The method for producing a biaryl compound according to [3], in which the acidic condition is set by an acid having a pKa of 0 or less.
[5] The method for producing a biaryl compound according to [3] or [4], in which the base is a hydroxide.
[6] The method for producing a biaryl compound according to any one of [1] to [5], in which a reaction temperature of the Diels-Alder reaction is 60°C or lower.
[7] The method for producing a biaryl compound according to any one of [1] to [6], in which the cross-coupling reaction is performed in a presence of a catalyst, and the catalyst includes a palladium catalyst and/or a nickel catalyst.
[8] The method for producing a biaryl compound according to any one of [1] to [7], in which a reducing agent is used in the cross-coupling reaction, and the reducing agent includes zinc.
[9] The method for producing a biaryl compound according to any one of [1] to [8], in which the biaryl compound includes an acene structure having 2 to 6 rings.
[10] The method for producing a biaryl compound according to [9], in which the acene structure having 2 to 6 rings is a naphthalene structure or an anthracene structure.

In the present specification, a numerical value range expressed using "to" means a range that includes the preceding and succeeding numerical values of "to" as the lower limit value and the upper limit value, respectively.

In the present invention, the term "X compound" is intended to encompass not only the compound "X" itself but also a compound in which "X" has a substituent, to the extent that the effects of the present invention are not impaired. For example, the term "o-benzyne compound" is intended to encompass not only the "o-benzyne" itself but also an o-benzyne having a substituent, to the extent that the effects of the present invention are not impaired.

In addition, the term "Y structure" is intended to encompass not only the chemical structure "Y" itself but also a structure in which "Y" has a substituent, to the extent that the effects of the present invention are not impaired.

In a case where there are a plurality of the above-described substituents, the substituents may be bonded to each other to form a ring within a range where the effect of the present invention is not impaired. The ring to be formed may be an aromatic ring (preferably a benzene ring), an aliphatic ring, or a fused ring (preferably a naphthalene ring). Therefore, for example, the "o-benzyne compound" includes a fused ring compound having 2 or more rings including an o-benzyne ring.

Examples of the above-described substituent (substituent that is not linked to each other to form a ring) include an alkyl group (preferably an alkyl group having 1 to 10 carbon atoms, more preferably an alkyl group having 1 to 6 carbon atoms, and still more preferably an alkyl group having 1 to 3 carbon atoms), an alkoxy group (preferably an alkoxy group having 1 to 10 carbon atoms, more preferably an alkoxy group having 1 to 6 carbon atoms, and still more preferably an alkoxy group having 1 to 3 carbon atoms), a halogen atom (for example, a fluorine atom, a chlorine atom, a bromine atom, or an iodine atom), an acyl group (preferably an acyl group having 2 to 10 carbon atoms, more preferably an acyl group having 2 to 6 carbon atoms, and still more preferably an acyl group having 2 or 3 carbon atoms), and an alkoxycarbonyl group (preferably an alkoxycarbonyl group having 2 to 10 carbon atoms, more preferably an alkoxycarbonyl group having 2 to 6 carbon atoms, and still more preferably an alkoxycarbonyl group having 2 or 3 carbon atoms). These substituents may further have a substituent.

In the present invention, the "fused ring compound" means a compound having a fused polycyclic structure in which two or more monocyclic rings are fused. The "fused ring compound" is preferably a compound having a linear fused polycyclic structure formed by fusion of two or more monocyclic rings (a compound in which the direction of fusion is linear).

In the present invention, the "biaryl compound" means a compound having a structure in which aromatic hydrocarbon rings are linked by a single bond. The aromatic hydrocarbon ring may be a monocyclic ring or a fused ring. In addition, a compound having a structure in which an aromatic hydrocarbon ring in a fused ring structure in which an aromatic hydrocarbon ring and an aliphatic ring are fused is linked by a single bond to another aromatic hydrocarbon ring or to an aromatic hydrocarbon ring in another fused ring structure in which an aromatic hydrocarbon ring and an aliphatic ring are fused is also the "compound having a structure in which aromatic hydrocarbon rings are linked by a single bond" (that is, the biaryl compound). In the present invention, the "biaryl compound" is not limited to a compound having one "structure in which aromatic hydrocarbon rings are linked by a single bond", and a compound having two or more "structures in which aromatic hydrocarbon rings are linked by a single bond" is also encompassed by the "biaryl compound".

With the method for producing a biaryl compound according to the present invention, it is possible to obtain a target biaryl compound without using, as a reaction substrate, an expensive reaction substrate such as a fused polycyclic aromatic boronic acid compound, in a case of introducing the fused polycyclic aromatic structure into a halogenated aryl compound to obtain the target biaryl compound.

### DESCRIPTION OF THE PREFERRED EMBODIMENTS

### [Method for producing biaryl compound]

In the method for producing a biaryl compound according to the embodiment of the present invention (hereinafter, also referred to as "production method according to the embodiment of the present invention"), first, an o-benzyne compound is generated from a precursor of the o-benzyne compound, and the o-benzyne compound and a furan compound are subjected to a Diels-Alder reaction to generate a fused ring compound (cycloadduct) including a 1,4-epoxy-1,4-dihydrobenzene ring. Furthermore, the fused ring compound and a halogenated aryl compound are subjected to a cross-coupling reaction to generate a target biaryl compound.

Each reaction constituting the production method according to the embodiment of the present invention will be described.

The precursor of the o-benzyne compound itself is known, and a compound that generates the o-benzyne compound by applying an external stimulus, a reagent, or the like can be widely used as the precursor of the o-benzyne compound in the present invention.

For example, an aromatic diazonium salt (a diazonium salt having a structure in which a carboxy group is bonded to a 1-position of a benzene ring and a diazo group is bonded to a 2-position of the benzene ring), obtained by allowing a nitrous acid compound that is a diazotizing agent to act on an anthranilic acid compound under an acidic condition, can be suitably used as the precursor of the o-benzyne compound. As the nitrous acid compound, an alkyl nitrite compound can be suitably used. As the alkyl nitrite compound, alkyl nitrite (preferably having 1 to 10 carbon atoms in the alkyl group, more preferably having 2 to 8 carbon atoms in the alkyl group, and still more preferably having 3 to 6 carbon atoms in the alkyl group) or the like can be used. For example, isopentyl nitrite is well known as the diazotizing agent.

In addition, it is known that a monohalogenobenzene compound, an o-dihalogenobenzene compound, a trifluoromethanesulfonic acid-2-(halogeno)phenyl compound, a trifluoromethanesulfonic acid-2-(trimethylsilyl)phenyl compound, or the like functions as the precursor of the o-benzyne compound, in addition to the above-described aromatic diazonium salt.

In a case where the above-described aromatic diazonium salt is used as the precursor of the o-benzyne compound, the o-benzyne compound can be efficiently generated by usually performing a treatment at a temperature of approximately 70°C or higher.

In addition, in a case where the above-described aromatic diazonium salt is used as the precursor of the o-benzyne compound, in the course of studying the present invention, the present inventors have found that, in a case where a base is added as described later, the o-benzyne compound is generated with high efficiency even at a lower temperature.

In a case where the above-described monohalogenobenzene compound is used as the precursor of the o-benzyne compound, for example, the o-benzyne compound can be generated by allowing lithium diisopropylamide to act, thereby dehydrogenating an ortho position relative to the carbon atom to which the halogen atom is bonded. The halogen atom included in the monohalogenobenzene compound is preferably a fluorine atom, a chlorine atom, a bromine atom, or an iodine atom, and more preferably a chlorine atom or a bromine atom.

In a case where the above-described o-dihalogenobenzene compound is used as the precursor of the o-benzyne compound, for example, the o-benzyne compound can be generated through a monolithiated species generated by action of alkyl lithium (preferably butyl lithium and more preferably n-butyl lithium). The halogen atom included in the o-dihalogenobenzene compound is preferably a chlorine atom, a bromine atom, or an iodine atom.

Even in a case where the above-described trifluoromethanesulfonic acid-2-(halogeno)phenyl compound is used as the precursor of the o-benzyne compound, for example, the o-benzyne compound can be generated through a monolithiated species generated by the action of alkyl lithium described above. The halogen atom included in the trifluoromethanesulfonic acid-2-(halogeno)phenyl compound is preferably a chlorine atom, a bromine atom, or an iodine atom.

In a case where the trifluoromethanesulfonic acid-2-(trimethylsilyl)phenyl compound is used as the precursor of the o-benzyne compound, for example, the o-benzyne compound can be generated through desilylation by action of tetrabutylammonium fluoride.

Among these, the aromatic diazonium salt obtained by diazotizing the anthranilic acid compound is preferable in that the anthranilic acid compound as a starting material is inexpensive, the o-benzyne compound can be generated with high efficiency, and as a result, a fused ring compound including a 1,4-epoxy-1,4-dihydrobenzene ring can be obtained with a high yield by the subsequent Diels-Alder reaction.

Therefore, a preferred embodiment of the production method according to the embodiment of the present invention includes, as a pre-step of generating the o-benzyne compound from the precursor of the o-benzyne compound, diazotizing the anthranilic acid compound under an acidic condition to obtain the aromatic diazonium salt. The diazotization reaction itself is well known, and in the present invention, a reaction temperature or a reaction time in a typical diazotization reaction can be adopted.

By diazotizing the anthranilic acid compound under an acidic condition to generate the aromatic diazonium salt, and then adding a furan compound and a base to subject the mixture to a Diels-Alder reaction, the o-benzyne compound can be generated with high efficiency without being subjected to a high temperature, and the Diels-Alder reaction can be efficiently carried out. This finding has not been previously known, and is found through the studying of the present inventors. The reason why the Diels-Alder reaction is enhanced by adding a base is not clear, but it is considered that one factor is that decarboxylation occurs by converting a carboxy group included in the aromatic diazonium salt into a carboxylate anion under a basic condition, thereby facilitating subsequent denitrogenation.

In a case where the diazotization reaction under an acidic condition and the Diels-Alder reaction to which a base is added are combined, a reaction temperature of the Diels-Alder reaction can be set to, for example, 60°C or lower, 55°C or lower, or 50°C or lower. Even in a case where the Diels-Alder reaction is carried out at a relatively low temperature as described above, a fused ring compound including a target 1,4-epoxy-1,4-dihydrobenzene ring can be obtained with a high yield. The reaction temperature is usually 30°C or higher, preferably 35°C or higher, and more preferably 40°C or higher. Therefore, in a case where the diazotization reaction under an acidic condition and the Diels-Alder reaction to which a base is added are combined, the reaction temperature of the Diels-Alder reaction is preferably 30°C to 60°C, more preferably 35°C to 55°C, and still more preferably 40°C to 50°C.

From the viewpoint of further suppressing side reactions in the Diels-Alder reaction, it is preferable to add a furan compound to the generated aromatic diazonium salt and then add a base.

In the above-described diazotization reaction under an acidic condition, it is preferable to establish the acidic condition using an acid (strong acid) having a pKa of 0 or less. In the present invention, values for the "pKa" may be a calculated value obtained using Advanced Chemistry Development (ACD/Labs) Software V11.02 (1994-2014 ACD/Labs) or a value described in the literature (for example, J. Phys. Chem. A 2011, 115, 6641-6645, and the like). Examples of the acid having a pKa of 0 or less include, for example, hydrochloric acid, sulfuric acid, methanesulfonic acid, p-toluenesulfonic acid, trifluoromethanesulfonic acid, trifluoroacetic acid, hydroiodic acid, hydrobromic acid, and nitric acid; and one kind or two or more kinds thereof can be used.

In addition, the base added in the above-described Diels-Alder reaction is preferably a strong base. With the strong base, the carboxy group included in the above-described aromatic diazonium salt is more reliably in a salt state, and the o-benzyne can be more efficiently generated. As the above-described base, a hydroxide is preferable. For example, potassium hydroxide, sodium hydroxide, ammonium hydroxide, lithium hydroxide, calcium hydroxide, barium hydroxide, or the like can be preferably used.

Preferred specific examples of the anthranilic acid compound from which the above-described aromatic diazonium salt is derived are shown below. Me represents methyl.

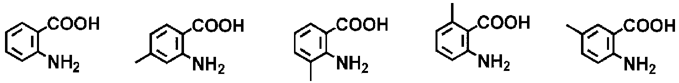

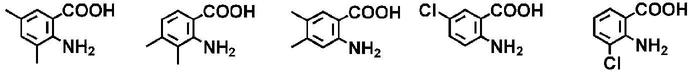

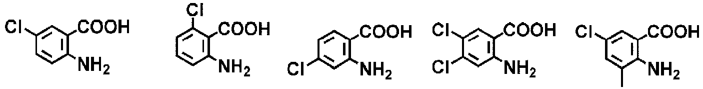

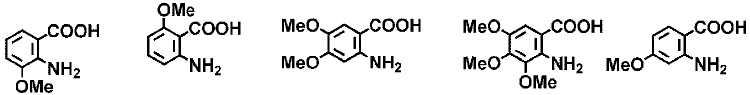

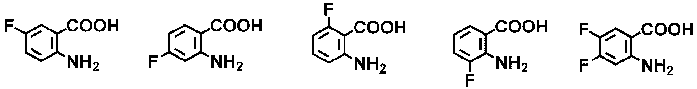

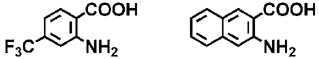

Preferred specific examples of the above-described furan compound are shown below.

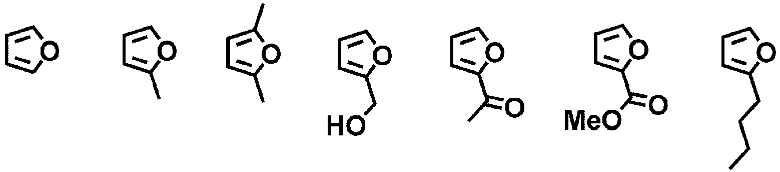

In the above-described Diels-Alder reaction, the amounts of the precursor of the o-benzyne compound and the furan compound used are such that a molar ratio [precursor/furan compound] is preferably 0.1/10 to 10/10, more preferably 0.5/10 to 8/10, still more preferably 1/10 to 6/10, and even more preferably 1.2/10 to 4/10.

In addition, a reaction time of the Diels-Alder reaction can be appropriately set according to the reaction temperature and the like. For example, in a case of a batch-type reaction, the reaction time can be set to approximately 10 minutes to 2 hours. In addition, in a case where a flow-type reaction described later is adopted, it is also possible to sufficiently carry out the reaction in a shorter time.

The above-described process from the generation of the o-benzyne compound to the Diels-Alder reaction may be performed in a batch mode or in a flow mode. In a case where the reaction liquid can maintain a solution state, it is preferable to employ a flow-type reaction because the fused ring compound including a 1,4-epoxy-1,4-dihydrobenzene ring can be continuously generated.

For example, in a case where 1-bromo-2-chlorobenzene is used as the precursor of the o-benzyne compound and furan is used as the furan compound, as an example, the following flow-type reaction system can be constructed.

An o-bromochlorobenzene solution and an n-butyllithium solution flowing through different flow paths are merged, and while the merged solution flows downstream, a bromine atom is substituted with a lithium atom to generate a monolithiated compound; and by merging the merged solution with a furan solution flowing through another flow path, it is possible, while this merged solution flows downstream, to cause the Diels-Alder reaction to occur between an o-benzyne compound generated from the monolithiated compound and furan. As necessary, by combining the combined liquid with a quenching agent such as methanol, target 1,4-epoxy-1,4-dihydronaphthalene can be obtained.

In the flow-type reaction system, a reaction temperature for monolithiation is controlled to a low temperature of, for example, -40°C or lower. In addition, in the Diels-Alder reaction step, it is preferable to raise the temperature. For example, it is preferable to carry out the Diels-Alder reaction by raising the temperature to approximately -30°C or higher.

As a solvent in the above-described reaction, an organic solvent capable of dissolving the above-described raw material or reagent can be appropriately used. For example, tetrahydrofuran, a hydrocarbon solvent, or the like can be used.

In addition, in a case where trifluoromethanesulfonic acid-2-(trimethylsilyl)phenyl is used as the precursor of the o-benzyne compound and furan is used as the furan compound, as an example, the following flow-type reaction system can be constructed.

A solution prepared by dissolving the trifluoromethanesulfonic acid-2-(trimethylsilyl)phenyl and the furan is allowed to flow through a flow path, and a tetra-n-butylammonium fluoride solution is allowed to flow through another flow path; and by merging these solutions, while the merged solution flows downstream, it is possible to cause the Diels-Alder reaction to occur between an o-benzyne compound generated through desilylation of the trifluoromethanesulfonic acid-2-(trimethylsilyl)phenyl and furan, thereby obtaining a target 1,4-epoxy-1,4-dihydronaphthalene.

In this flow type reaction system, a reaction temperature of the Diels-Alder reaction is preferably controlled to, for example, approximately -40°C to -10°C.

As a solvent in the above-described reaction, an organic solvent capable of dissolving the above-described raw material or reagent can be appropriately used. For example, tetrahydrofuran, a hydrocarbon solvent, or the like can be used.

Subsequently, a cross-coupling reaction between the fused ring compound including a 1,4-epoxy-1,4-dihydrobenzene ring (sometimes simply referred to as "fused ring compound") obtained by the above-described Diels-Alder reaction and a halogenated aryl compound will be described.

The above-described cross-coupling reaction is usually performed in the presence of a catalyst. Examples of the catalyst include a palladium (Pd) catalyst, a nickel (Ni) catalyst, a copper (Cu) catalyst, and an iron (Fe) catalyst. Among these, a Pd catalyst and/or a Ni catalyst is suitable. As the Pd catalyst, catalysts used in various coupling reactions can be used, for example, bis(triphenylphosphine)palladium(II) dichloride, palladium(II) chloride, palladium(II) acetate, tetrakis(triphenylphosphine)palladium(0), tris(dibenzylideneacetone)palladium(0), [1,2'-bis(diphenylphosphino)ferrocene]dichloropalladium(II), and the like. In addition, as the Ni catalyst, catalysts used in various coupling reactions can be used, for example, bis(triphenylphosphine)nickel(II) dichloride, nickel(II) chloride, [1,3-bis(diphenylphosphino)propane]dichloronickel(II), [1,2-bis(diphenylphosphino)ethane]dichloronickel(II), nickel(II) acetate, [1,1'-bis(diphenylphosphino)ferrocene]dichloronickel(II), and the like.

One kind of the catalyst may be used, or two or more kinds thereof may be used in combination.

The above-described cross-coupling reaction is usually performed using a reducing agent. Examples of the reducing agent include zinc, magnesium, diisobutylaluminum hydride, n-butyllithium, and triphenylphosphine. Among these, zinc is suitable from the viewpoint of improving the yield.

One kind of the reducing agent may be used, or two or more kinds thereof may be used in combination.

The above-described cross-coupling reaction is preferably performed in the presence of a base. As the base, a base that can be used in a general cross-coupling reaction can be widely used. From the viewpoint of reaction efficiency, an organic amine compound is preferable. For example, trialkylamine (the number of carbon atoms in an alkyl group is preferably 1 to 10, more preferably 1 to 8, still more preferably 2 to 6, and even more preferably 2 to 4), triethylamine, diisopropylethylamine, N-methylmorpholine, dimethylbenzylamine, and the like are suitable bases; and among these, triethylamine can be suitably used.

In the above-described cross-coupling reaction, a ratio of amounts of the above-described fused ring compound and the halogenated aryl compound can be determined approximately based on stoichiometry. For example, in a case where the number of halogen atoms in the halogenated aryl compound is 1, the amounts of the fused ring compound and the halogenated aryl compound used are such that a molar ratio [fused ring compound/halogenated aryl compound] is preferably 0.1/1 to 10/1, more preferably 0.5/1 to 5/1, and still more preferably 1/1 to 2/1.

A reaction temperature of the above-described cross-coupling reaction is not particularly limited as long as the desired reaction proceeds. For example, the reaction temperature can be set to 60°C to 150°C, preferably 80°C to 130°C, and more preferably 100°C to 120°C.

In addition, a reaction time of the above-described cross-coupling reaction is not particularly limited as long as the desired reaction proceeds. For example, the reaction time can be set to 0.5 to 10 hours, may be set to 1 to 8 hours, and can also preferably be set to 2 to 6 hours.

After the above-described cross-coupling reaction is completed, the target biaryl compound can be obtained in the reaction solution. The biaryl compound can be subjected to treatments such as washing, concentration, and purification as necessary.

The biaryl compound obtained by the production method according to the embodiment of the present invention has a fused polycyclic aromatic structure derived from a fused ring compound including a 1,4-epoxy-1,4-dihydrobenzene ring. The fused polycyclic aromatic structure is preferably an acene structure having 2 to 6 rings, and among these, a naphthalene structure or an anthracene structure is preferable.

Preferred specific examples of the biaryl compound obtained by the production method according to the embodiment of the present invention are shown below, but the present invention is not limited thereto except as defined in the present invention. Me represents methyl and Et represents ethyl.

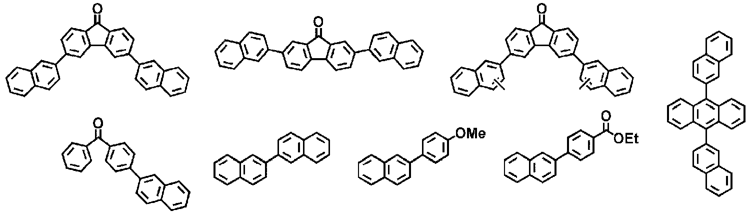

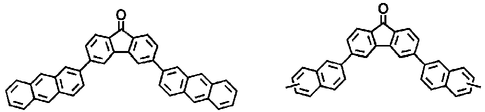

The present invention will be described in more detail based on Examples; however, the present invention should not be construed as being limited to these Examples except as defined in the present invention.

### Examples

### [Example 1]

1,4-Epoxy-1,4-dihydronaphthalene (referred to as "epoxydihydronaphthalene 1") was prepared according to the following reaction scheme.

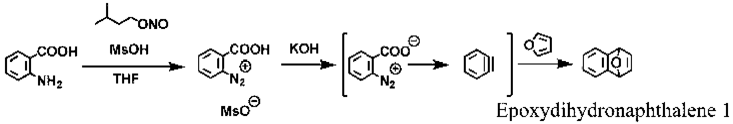

### The preparation of the epoxydihydronaphthalene 1 will be specifically described.

A calcium chloride tube was attached to a 500 mL three-necked flask, and 200 mL of anhydrous tetrahydrofuran (anhydrous THF) and 5 g of anthranilic acid were charged into the three-necked flask. After dissolving the anthranilic acid, while maintaining the solution at 0°C to 10°C, 3.85 g of methanesulfonic acid (MsOH) was added dropwise into the three-necked flask, and then 6.41 g of isopentyl nitrite was added dropwise into the three-necked flask. Thereafter, the temperature was raised to room temperature (25°C), and the mixture was stirred for 30 minutes. In this way, an aromatic diazonium salt, which is a precursor of benzyne, was obtained in the solution. It was confirmed by thin-layer chromatography (TLC) that the anthranilic acid disappeared.

12.4 g of furan was put into a three-neck flask, and then 8.86 g of a potassium hydroxide solution (solvent: methanol) having a concentration of 30% by mass was charged into the three-neck flask. By raising the temperature to 45°C and stirring for 1 hour, a Diels-Alder reaction proceeded between the o-benzyne generated from the aromatic diazonium salt and furan, and the epoxydihydronaphthalene 1, which is the "fused ring compound including a 1,4-epoxy-1,4-dihydrobenzene ring," was generated in the reaction solution. Thereafter, the mixture was cooled to 10°C to 20°C, 50 mL of a 20% by mass sodium chloride aqueous solution was added dropwise to the three-neck flask, and then the reaction solution was transferred to a separating funnel and the aqueous layer was removed. 500 µL of dimethylformamide (DMF) and 10 g of magnesium sulfate were added to the organic layer to dry the organic layer, magnesium sulfate was filtered and removed, and the obtained solution was concentrated to obtain the epoxydihydronaphthalene 1 (yield: 80%, solid content: 45% by mass). It was confirmed by high-performance liquid chromatography (HPLC), nuclear magnetic resonance (NMR), and liquid chromatography/mass spectrometry (LC/MS) that the epoxydihydronaphthalene 1 was obtained.

A biaryl compound 1 having the following structure was prepared according to the following reaction scheme.

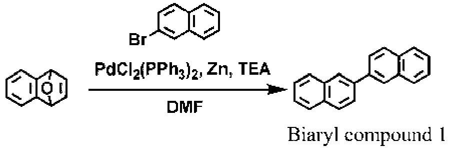

The preparation of the biaryl compound 1 will be specifically described.

Into a 50 mL three-necked flask, 0.22 g of 2-bromonaphthalene as a halogenated aryl compound, 0.41 g of the epoxydihydronaphthalene 1 (45% by mass solution) prepared as described above (1.2 equivalents with respect to 2-bromonaphthalene), 38.7 mg of zinc as a reducing agent, 20.8 mg of bis(triphenylphosphine)palladium(II) dichloride (PdCl₂(PPh₃)₂) as a catalyst, and 4 mL of DMF were charged, and then the atmosphere in the three-necked flask was replaced with nitrogen. Thereafter, 0.41 mL of triethylamine (TEA) as a base was added to the three-neck flask, and the mixture was stirred at 110°C for 4 hours to carry out a cross-coupling reaction. In this way, a target biaryl compound 1 was obtained (HPLC production rate: 80%).

The "HPLC production rate" was calculated according to the following expression based on a calibration curve prepared using a standard of the biaryl compound 1 with the following HPLC apparatus under the following operating conditions.
Apparatus: prominence series (manufactured by Shimadzu Corporation)
Detector: UV/VIS light absorption detector SPD-20A, 210 nm or 254 nm
Column: Shiseido CAPCELL PAK C1, TYPE: UG120, 5 µm, SIZE: 4.6 φ × 250 mm Column oven: 40°C
Mobile phase A: water, 0.1% phosphoric acid, 0.1% triethylamine
Mobile phase B: acetonitrile
Mobile phase concentration gradient: 0.01 min (B: 30%), 15.00 min (B: 100%), 15.01 min (B: 30%), 20.00 min (B: 30%) 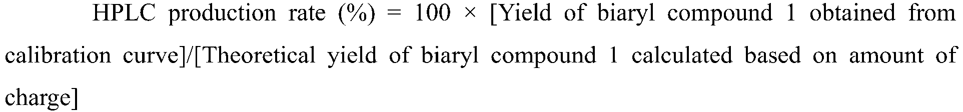 HPLC production rate (%) = 100 × [Yield of biaryl compound 1 obtained from calibration curve]/[Theoretical yield of biaryl compound 1 calculated based on amount of charge]

### [Example 2]

A biaryl compound 2 having the following structure was obtained in the same manner as in Example 1, except that, in the preparation of the biaryl compound 1 in Example 1, 4-bromoanisole was used as described below in place of the 2-bromonaphthalene (HPLC production rate: 50%).

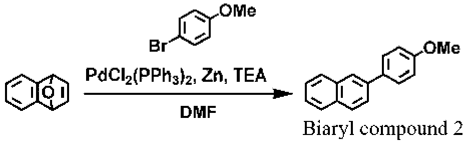

### [Example 3]

A biaryl compound 3 having the following structure was obtained in the same manner as in Example 1, except that, in the preparation of the biaryl compound 1 in Example 1, ethyl 4-bromobenzoate was used as described below in place of the 2-bromonaphthalene (HPLC production rate: 68%). Et represents ethyl.

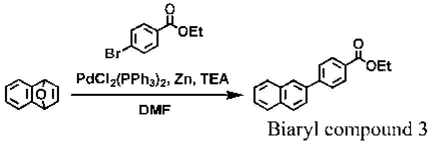

### [Example 4]

A biaryl compound 4 having the following structure was obtained in the same manner as in Example 1, except that, in the preparation of the biaryl compound 1 in Example 1, 9,10-dibromoanthracene was used as described below in place of the 2-bromonaphthalene, and the epoxydihydronaphthalene 1 was used in an amount of 2.4 equivalents with respect to 9,10-dibromoanthracene (HPLC production rate: 40%).

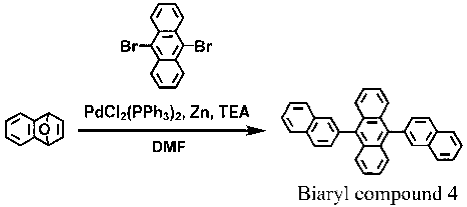

### [Example 5]

A biaryl compound 5 having the following structure was obtained in the same manner as in Example 1, except that, in the preparation of the biaryl compound 1 in Example 1, 3,6-dibromofluorenone was used instead of 2-bromonaphthalene, and the epoxydihydronaphthalene 1 was used in an amount of 2.4 equivalents with respect to 3,6-dibromofluorenone (HPLC production rate: 93%).

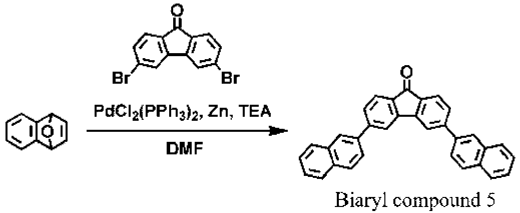

### [Example 6]

As shown in the following reaction scheme, 6-methyl-1,4-epoxy-1,4-dihydronaphthalene (referred to as "epoxydihydronaphthalene 2") was obtained in the same manner as in the preparation of the epoxydihydronaphthalene 1 of Example 1, except that 2-amino-5-methylbenzoic acid was used in place of the anthranilic acid (yield: 61%, solid content: 49% by mass).

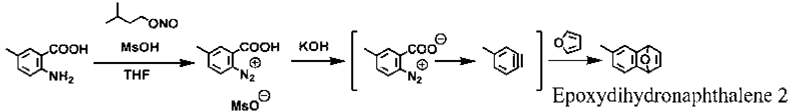

As shown in the following reaction scheme, a methylated biaryl compound mainly containing a biaryl compound 6 having the following structure was obtained in the same manner as in Example 5, except that, in the preparation of the biaryl compound 5 in Example 5, the epoxydihydronaphthalene 2 prepared above was used in place of the epoxydihydronaphthalene 1 (HPLC production rate: 28%).

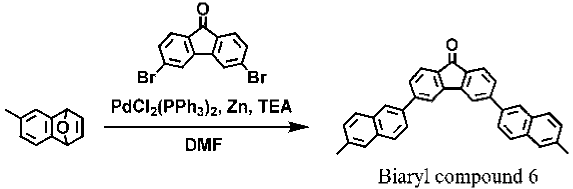

### [Example 7]

As shown in the following reaction scheme, 1-methyl-1,4-epoxy-1,4-dihydronaphthalene (referred to as "epoxydihydronaphthalene 3") was obtained in the same manner as in the preparation of the epoxydihydronaphthalene 1 of Example 1, except that 2-methylfuran was used in place of the furan (yield: 28%, solid content: 18% by mass).

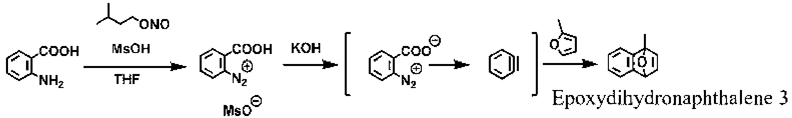

As shown in the following reaction scheme, a methylated biaryl compound mainly containing a biaryl compound 7 having the following structure was obtained in the same manner as in Example 5, except that, in the preparation of the biaryl compound 5 in Example 5, the epoxydihydronaphthalene 3 prepared above was used in place of the epoxydihydronaphthalene 1 (HPLC production rate: 76%).

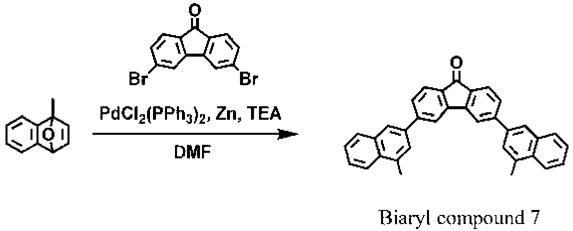

### [Example 8]

A biaryl compound 8 having the following structure was obtained in the same manner as in Example 5, except that, in the preparation of the biaryl compound 5 in Example 5, 2,7-dibromofluorenone was used as described below in place of the 3,6-dibromofluorenone, and the epoxydihydronaphthalene 1 was used in an amount of 2.4 equivalents with respect to 2,7-dibromofluorenone (HPLC production rate: 80%).

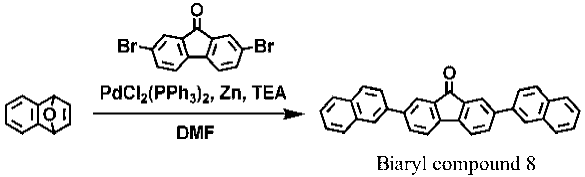

### [Example 9]

A biaryl compound 9 having the following structure was obtained in the same manner as in Example 1, except that, in the preparation of the biaryl compound 1 of Example 1, 4-bromobenzophenone was used as described below in place of the 2-bromonaphthalene (HPLC production rate: 91%).

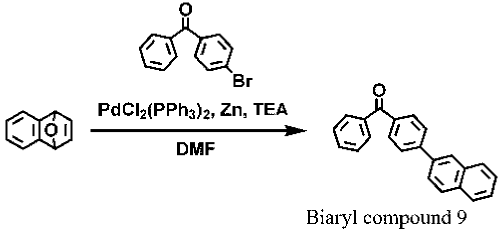

### [Example 10]

In the preparation of the biaryl compound 1 in Example 1, the biaryl compound 1 was obtained by changing the catalyst as described below.

Into a 50 mL three-necked flask, 122.5 mg of 2-bromonaphthalene as a halogenated aryl compound, 0.41 g of the epoxydihydronaphthalene 1 (45% by mass solution) prepared as described above, 100.6 mg of zinc, 38.7 mg of bis(triphenylphosphine)nickel(II) dichloride (NiCl₂(PPh₃)₂), 116.4 mg of triphenylphosphine, and 4 mL of DMF were charged, and then the atmosphere in the three-necked flask was replaced with nitrogen. Thereafter, 0.41 mL of triethylamine (TEA) was added to the three-necked flask, and the mixture was stirred at 110°C for 4 hours to carry out a cross-coupling reaction. Ethyl acetate was added to the reaction solution, and the organic layer was washed with 1 N hydrochloric acid and brine. The organic layer was dried over magnesium sulfate, concentrated, and purified by column chromatography to obtain the target biaryl compound 1 (HPLC production rate: 80%).

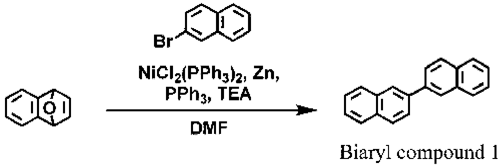

### [Example 11]

The biaryl compound 1 was obtained in the same manner as in Example 10, except that, in the preparation of the biaryl compound 1 in Example 10, magnesium was used as a reducing agent in place of the zinc, and triphenylphosphine was not used (HPLC production rate: 50%).

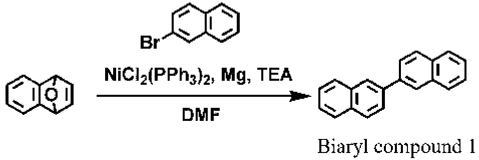

### [Example 12]

The biaryl compound 1 was obtained in the same manner as in Example 10, except that, in the preparation of the biaryl compound 1 in Example 10, diisobutylaluminum hydride (DIBAH) was used in place of the zinceas a reducing agent in place of the zinc, and triphenylphosphine was not used (HPLC production rate: 30%).

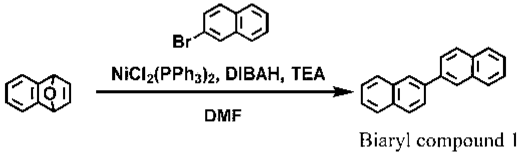

### [Example 13]

The biaryl compound 1 was obtained in the same manner as in Example 10, except that, in the preparation of the biaryl compound 1 in Example 10, n-butyllithium was used as a reducing agent in place of the zinc, and triphenylphosphine was not used (HPLC production rate: 20%).

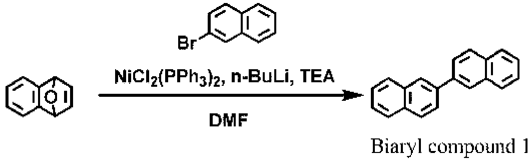

### [Example 14]

The biaryl compound 1 was obtained in the same manner as in Example 10, except that, in the preparation of the biaryl compound 1 in Example 10, potassium phosphate was used as a base in place of the triethylamine (HPLC production rate: 20%).

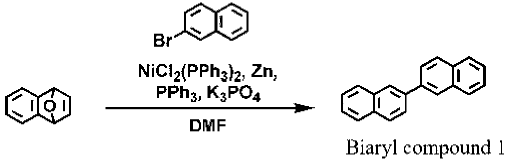

### [Example 15]

The biaryl compound 1 was obtained in the same manner as in Example 10, except that, in the preparation of the biaryl compound 1 in Example 10, sodium carbonate was used as a base in place of the triethylamine (HPLC production rate: 11%).

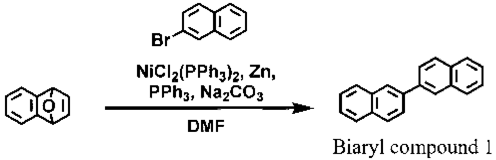

### [Example 16]

The biaryl compound 1 was obtained in the same manner as in Example 10, except that, in the preparation of the biaryl compound 1 in Example 10, potassium hydroxide was used as a base in place of the triethylamine (HPLC production rate: 20%).

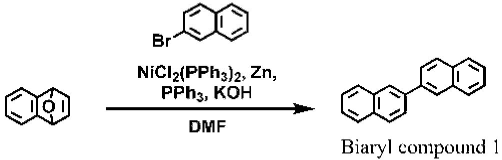

### [Example 17]

As shown in the following reaction scheme, 1,4-epoxy-1,4-dihydroanthracene (referred to as "epoxydihydroanthracene") was prepared in the same manner as in the preparation of the epoxydihydronaphthalene 1 in Example 1, except that, in the preparation of the epoxydihydronaphthalene 1 in Example 1, 3-amino-2-naphthoic acid was used in place of the anthranilic acid (yield: 50%, solid content: 40% by mass).

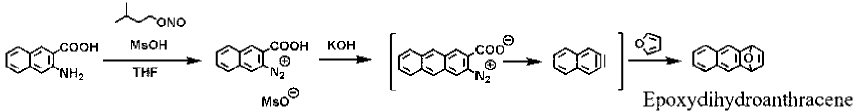

As shown in the following reaction scheme, a biaryl compound 10 having the following structure was prepared in the same manner as in Example 1, except that the epoxydihydroanthracene prepared as described above was used in place of the epoxydihydronaphthalene 1 (HPLC production rate: 50%).

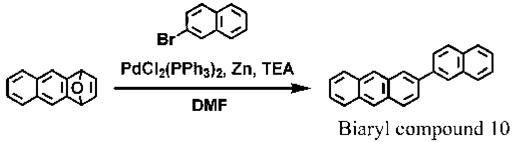

### [Example 18]

A biaryl compound 11 having the following structure was obtained in the same manner as in Example 1, except that, in the preparation of the biaryl compound 1 of Example 1, 5-bromo-1,10-phenanthroline was used as described below in place of the 2-bromonaphthalene (HPLC production rate: 17%).

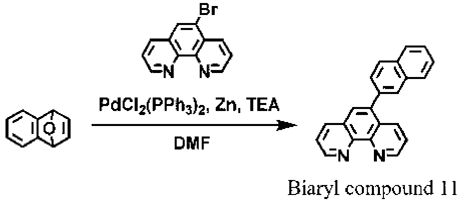

From the above results, it was found that the target biaryl compound was obtained by the production method according to the embodiment of the present invention (novel synthetic route).

In addition, from the comparison between Example 1 and Example 10, it was found that the target biaryl compound was obtained with a high yield using either the Pd catalyst or the Ni catalyst, as the catalyst for the cross-coupling reaction.

In addition, from the comparison between Example 10 and Examples 11 to 13, it was found that the reducing agent used in the cross-coupling reaction affected the yield of the target biaryl compound, and that, in particular, using zinc as the reducing agent increased the yield of the target biaryl compound.

In addition, from the comparison between Example 10 and Examples 14 to 16, it can also be seen that the base used in the cross-coupling reaction affected the yield of the target biaryl compound.

The present invention has been described using the embodiments. However, unless specified otherwise, any of the details of the above description is not intended to limit the present invention and can be construed in a broad sense within a range not departing from the concept and scope of the present invention disclosed in the accompanying claims.

This application claims priority based on JP2023-202640 filed on November 30, 2023, the contents of which are incorporated herein by reference as part of the present specification.

## Claims

1. A method for producing a biaryl compound, comprising:
generating an o-benzyne compound from a precursor of the o-benzyne compound;
subjecting the o-benzyne compound and a furan compound to a Diels-Alder reaction to generate a fused ring compound including a 1,4-epoxy-1,4-dihydrobenzene ring; and
subjecting the fused ring compound and a halogenated aryl compound to a cross-coupling reaction to generate a biaryl compound.

2. The method for producing a biaryl compound according to claim 1, wherein the precursor of the o-benzyne compound is an aromatic diazonium salt.

3. The method for producing a biaryl compound according to claim 1, wherein the method includes diazotizing an anthranilic acid compound under an acidic condition to obtain an aromatic diazonium salt that is the precursor of the o-benzyne compound, and adding a base to a reaction system for the Diels-Alder reaction.

4. The method for producing a biaryl compound according to claim 3, wherein the acidic condition is set by an acid having a pKa of 0 or less.

5. The method for producing a biaryl compound according to claim 4, wherein the base is a hydroxide.

6. The method for producing a biaryl compound according to claim 1, wherein a reaction temperature of the Diels-Alder reaction is 60°C or lower.

7. The method for producing a biaryl compound according to claim 1, wherein the cross-coupling reaction is performed in a presence of a catalyst, and the catalyst includes a palladium catalyst and/or a nickel catalyst.

8. The method for producing a biaryl compound according to claim 7, wherein a reducing agent is used in the cross-coupling reaction, and the reducing agent includes zinc.

9. The method for producing a biaryl compound according to any one of claims 1 to 8, wherein the biaryl compound includes an acene structure having 2 to 6 rings.

10. The method for producing a biaryl compound according to claim 9, wherein the acene structure having 2 to 6 rings is a naphthalene structure or an anthracene structure.
